# EUROPEAN PATENT APPLICATION

(11) **EP 3 950 955 A1**
(43) Date of publication of application: **09.02.2022**
(21) Application number: 20189814.5
(22) Date of filing: 06.08.2020
(51) Int. Cl.: C12Q 1/6804, C12Q 1/6853

(54) **MULTIANALYTE ASSAY FOR THE SIMULTANEOUS DETECTION OF NUCLEIC ACID AND ANALYTES**

(71) Applicant: Albert-Ludwigs-Universität Freiburg, 79098 Freiburg (DE); Hahn-Schickard-Gesellschaft für angewandte Forschung e.V., 78052 Villingen-Schwenningen (DE)
(72) Inventor: FRÜH, Susanna Maria, 79106 Freiburg (DE); BRUNAUER, Anna, 79111 Freiburg (DE)
(74) Representative: Hertin und Partner Rechts- und Patentanwälte PartG mbB

(57) **Abstract**

The present invention relates to a method and a kit to detect the presence or absence of at least two different target molecules form one sample, wherein at least one target molecule is a target analyte of interest and at least one other target molecule is a target nucleic acid of interest, wherein the method combines performing an isothermal amplification reaction, wherein the target nucleic acid or its amplicon is labeled with at least two affinity labels and a ligand binding assay, wherein affinity molecules are used, which can capture and detect the presence of target analytes and/or labeled target nucleic acid via signal generation. The invention also relates to the use of this method or kit in various fields.

## Description

### Background of the invention

### Technical field

For the detection of molecules (e.g. nucleic acids, analytes) several detection methods are available in the state of the art. Each of these methods is specialized and optimized for a specific biomolecule class (e.g. PCR or isothermal amplification methods for nucleic acid detection or immunoassays for protein detection).

In many applications, it is advantageous to detect several classes of molecules simultaneously in a single step. Arguments have been made for the need of multiplex detection of nucleic acids and analytes in a single step, however, approaches need to face several challenges. They need to consider the concentration differences between nucleic acids and analytes in biological samples. Physiological relevant concentration ranges of analytes and nucleic acids may not be measured simultaneously. Thus, amplification of the target nucleic acid is required. However, the required amplification conditions often lead to analyte changes e.g. protein denaturation (e.g. heating steps in a PCR reaction). Once the analyte has an e.g. non-native structure it cannot be detected by standard immunological assays.

### Discussion of related art

First approaches for the simultaneous detection of analytes and nucleic acids have been developed. However, these approaches still have limitations. One approach includes the detection of nucleic acids via hybridization to probes and the simultaneous detection of the analyte via antigen-antibody interaction (Mohan et al. 2011; Mao et al. 2014; Scott et al. 2017). Both reactions require different conditions - which interfere with each other. Whereas, analytes are detected in buffered systems, the hybridization reaction requires high salt concentration and relative high temperatures. The development of probes that hybridize at 37°C to the target nucleic acid allow the simultaneous detection of the analyte, but still lead to a weak signal-to-noise ratio (Mohan et al. 2011). Furthermore, some approaches analyze the target molecules in a sequential approach (Mohan et al. 2011) and/or require a separate sample preparation step (Mohan et al. 2011; Scott et al. 2017).

Several approaches transform analyte information into nucleic acid information by using analyte detecting molecules like aptamers or antibodies linked to oligonucleotides. Subsequent amplification of the aptamer or the oligonucleotide (linked to the antibody) together with the target nucleic acid allows the simultaneous detection of the target molecules. Thus, the same measuring principle is used for the simultaneous detection of nucleic acids and analytes. However, the reduced stability of aptamers - compared to antibodies - and selectivity in complex and crude sample matrices hampers the widespread application of aptamers for analyte detection. On the other side, conjugation of oligonucleotides to the analyte detecting antibody can be a time-consuming process. The current approaches use different strategies to amplify the target analyte-binding oligonucleotide, including two-ended adapter ligation method, branched-chain assay, and proximity extension assay. All these approaches require the design of several primers and/or probes additionally to primers and probes for the detection of the target nucleic acid. This increases the complexity of the system, can add to the costs and might lead to a high background due to primer/probe dimer formation.

In the state of the art Mohan et al. 2011 describes an electrochemical sensor platform that can be adopted for both nucleic acid and protein detection. The protein detection is based on antibody-antigen reaction whereas, the nucleic acid detection is based on a hybridization reaction. To facilitate an integration of the nucleic acid detection with the protein immunoassay they developed probes optimized for hybridization, at 37°C. The detection is based on a horseradish peroxidase (HRP) redox reaction. This approach is based on two different measuring principles. Nucleic acids are detected via hybridization of probes, whereas, proteins are detected via an antigen-antibody reaction. In general, these two measuring principles require different assay conditions. However, the authors developed a protein and nucleic acid assay that share similar parameters. This compromise bears the risk to affect the sensitivity and specificity of one or both assays. Furthermore, the approach does not consider concentration differences between proteins and nucleic acids in biological samples. Physiological relevant concentration ranges of proteins and nucleic acids cannot be measured simultaneously. Additionally, the approach also requires separate sample preparation and separate addition of the protein or nucleic acid sample to the sensor.

Mao et al. 2014 describes a lateral flow device for the simultaneous detection of nucleic acids and proteins. Two individual reactions are performed on the lateral flow strip. The protein is detected via sandwich-type immunoreaction whereas, the nucleic acid detection is based on a DNA hybridization reaction. Target nucleic acid and protein are applied simultaneously to the sample application zone and bind to their corresponding test line. The target molecules are detected via gold nanoparticles functionalized with detection probes or detection antibodies. This approach uses two different measuring principles. Nucleic acids are detected via hybridization of probes, whereas, proteins are detected via an antigen-antibody reaction. This results in a low sensitivity of the assay. Furthermore, the approach uses quite short single stranded DNA and thus is not suitable for longer double stranded DNAs in biological samples. The nucleic acid is not amplified prior detection, thus only very high amounts of nucleic acids can be detected, which might not be physiologically relevant.

Scott et al. 2017 describes a combinatorial assay that provides the ability to simultaneously detect proteins and nucleic acids on a microarray. The detection is based on an antibody-antigen reaction and a DNA hybridization reaction of the detection of the target nucleic acid to the microarray. The array is functionalized with a capture probe for nucleic acid detection and a capture antibody for protein detection. Prior to the assay the 3' terminal of the target nucleic acid is biotinylated and both - target protein and biotinylated target nucleic acid - are added to the array. After binding to the corresponding areas of the microarray a biotinylated antibody is added and binds to the target protein. Subsequently, the target molecules are detected via Biotin-PEG-Linked gold nanoparticles.

This approach uses two different measuring principles. Nucleic acids are detected via hybridization of probes, whereas, proteins are detected via an antigen-antibody reaction. In general, these two measuring principles require different assay conditions for optimal results. Furthermore, the approach requires separate sample preparation. The 3' terminal of the nucleic acid needs to be biotinylated with a biotinylation kit prior detection, which adds an additional sample preparation step. Biotinylated non-target nucleic acids can also bind to the Biotin-PEG-Linked gold nanoparticles and thus occupy binding sites and might lead to a high background signal. Furthermore, the approach is only suitable for short single-stranded RNA or DNA sequences and only very high amounts of nucleic acids can be detected, because the nucleic acid is not amplified. To hybridize the biotinylated target nucleic acid to the detection probe the sample is incubated for 2 hours at 45 °C with the microarray. These assays conditions are suboptimal for protein detection - that is binding simultaneously to its capture antibody -and might lead to protein degradation.

US8431367B2 describes a method for the detection of a target nucleic acid and a target protein in a single assay using the aptamer technology. Thus, it is possible to amplify target nucleic acid and aptamer in a single reaction. Subsequently, the amplified target nucleic acid and aptamer can be detected via standard nucleic acid detection techniques such as sequencing of fluorescence labeling methods. Detection of the aptamer therefore indicates the presence of the target protein in the sample. In general, aptamers are less established than mono- or polyclonal antibodies and their overall negative charge, making them hydrophilic, and they are rapidly degraded by nucleases. Furthermore, aptamers have only four nucleic acid building block (compared to 22 amino acid building block of proteins), limiting their diversity of possible secondary and tertiary structures. Additionally, their custom synthesis adds to the cost. Especially the aptamer stability and selectivity in complex and crude sample matrices hampers the widespread application of aptamers for protein detection.

WO2019157445A1 describes the simultaneous, multiplexed detection and quantification of proteins and/or nucleic acids expression in a used-defined region of a tissue, cell or subcellular structure using existing sequencing methods. Target analytes are detected using probes in a method described as "two-ended adapter ligation method". The specific probe contains a target binding domain and an identifier oligonucleotide which identifies the target analyte bound to the target binding domain. Further, the probe might contain a photo-cleavable linker between the target binding domain and the identifier oligonucleotide. By applying light of a sufficient wavelength the linker is cleaved and thus releasing the identifier oligonucleotide. After collecting the released identifier oligonucleotides a first nucleic acid probe and a second nucleic acid probe are hybridizing to the released identifier oligonucleotide. These nucleic acid probes contain different domains. The first nucleic acid probe contains a sequence complementary to a part of the identifier oligonucleotide, a sequence comprising a unique molecular identifier and an amplification primer binding site. The second nucleic acid probe contains a sequence complementary to a part of the identifier oligonucleotide and a second amplification primer binding site. The hybridized nucleic acid probes are ligated and subsequently the ligation product is amplified. Using existing sequencing methods, the amplified ligation product is analyzed and the target analyte is identified.

The main drawback is, that this is a method, which relies on single molecule counting. Single molecule counting requires either complex, expensive, and sensitive readout equipment like specialized microscopes or if low-cost optics are used complex, long (barcoded) primes and probes have to be designed for every target molecule, which is again very costly and requires extensive assay design. Furthermore, the high background due to primer/probe dimer formation is an issue.

US20120004132A1 describes a method for the detection of multiple nucleic acids and proteins using a branched-chain based assay. In general, this system is very complex and requires several primers and probes, which adds to the costs of the assay. Furthermore, the branched-chain assay employs linear signal amplification rather than exponential amplification of the target molecule.

US10214773B2 describes a method for the detection of nucleic acids and proteins using a proximity extension assay. This approach uses an antibody pair that binds in close proximity to the target protein. Each antibody is conjugated to a nucleic acid that hybridizes to the nucleic acid of the other antibody. Thus, this approach is highly depending upon antibody quality - both antibodies need to bind to the target with the same efficiency, but to different epitopes in close proximity. Hence, small molecules such as drugs or toxins or smaller proteins cannot be detected. Furthermore, the conjugation of oligonucleotides to antibodies can be time consuming. Additionally, the nonspecific ligation of nucleotides and the formation of dimers can lead to a high background signal.

US20180208975A1 and US20180251825A1 disclose a similar approach to detect proteins and nucleic acid in a sample. The protein is recognized by a protein-binding molecule (e.g. antibody) conjugated to a DNA barcode. This oligonucleotide sequence contains a primer for amplification and sequencing (PCR handle), a protein identification sequence that identifies the protein-binding molecule, and a universal linker sequence that hybridizes to a sequence in the detection probe. The approaches differ in how the protein-binding molecule is conjugated to the DNA barcode. US20180251825A1 uses streptavidin-biotin interaction to link the DNA barcodes to e.g. antibodies. Whereas, in US20180208975A1 the protein binding molecule is covalently bound to the aminated DNA barcode. However, the universal linker sequence of the DNA barcode and the nucleic acid hybridize to the corresponding protein detection probe and to the nucleic acid detection probe. Subsequently, the sequences are amplified and sequenced to detect the signals for the analytes.

Both approaches are used for single cell analysis. The main limitation is the epitope location of the antibody, which is currently restricted to the cell surface and thus intracellular proteins are not detected. To capture the individual cells a single-cell suspension is required. This step often uses enzymatic treatments to break down tissues which may affect the transcriptional profile of the cell and low RNA quality. Furthermore, the conjugation of oligonucleotides to antibodies can be time consuming.

All in all, the current approaches for the simultaneous detection of analytes and nucleic acids have limitations regarding sensitivity, reproducibility, signal-to-noise ratio, or require separated sample preparation for the different target molecules.

Therefore, a need exists to develop a multianalyte approach that avoids the drawback of the state-of-the-art. Additionally, a reaction protocol is beneficial, which that can be implemented into different applications and platforms.

### DISCLOSURE OF THE INVENTION

This problem is solved by the features of the independent claims. Preferred embodiments of the present invention are provided by the dependent claims.

In a first embodiment the invention relates to a method to detect the presence or absence of at least two different target molecules form one sample, wherein at least one target molecule is a target analyte of interest and at least one other target molecule is a target nucleic acid of interest, the method comprising
- performing an isothermal amplification reaction, comprising
   ∘ contacting a sample to be analyzed for the presence or absence of at least one target nucleic acid and/or at least one target analyte to at least one set of amplification primers, comprising at least two amplification primers,
   ∘ wherein the two amplification primers can hybridize with the target nucleic acid,
   ∘ wherein at least one of the amplification primers comprises a first affinity label,
   ∘ wherein a second affinity label is provided in a way that in can be incorporated into an amplicon of the target nucleic acid,
- simultaneously performing a ligand binding assay, wherein affinity molecules are used, which can capture and detect the presence of target analytes and/or labeled target nucleic acid via signal generation,
- detecting the presence or absence of said target analytes and/or target nucleic acids.

In general, most physiological relevant concentration ranges of analytes and nucleic acids cannot be measured simultaneously. However, the invention uses a nucleic-acid-to-affinity-ligand-transformation which amplifies the target nucleic acid and functionalizes the amplicon with labels. Thus, a simultaneous detection of physiological relevant concentration ranges of different target molecule classes is now possible.

It is preferred that the method of the invention is used to detect the presence of at least two different classes of target molecules.

The invention presents a novel solution for the simultaneous detection of nucleic acids and analytes in one go from a sample by functionalizing amplified nucleic acid sequences with labels (Nucleic-acid-to-affinity-ligand-transformation) and detecting labeled target nucleic acids and target analytes with the same measuring principle. This is achieved by (1) using analyte compatible isothermal amplification for the introduction of labels into the target nucleic acids, and (2) detecting the target molecules via ligand binding assay.

To enable simultaneous detection of target nucleic acids and target analytes with the same measuring principle the information of the different target molecules needs to be transformed into a single information format. For example: the information of a certain analyte e.g. protein is transformed into nucleic acid information. This for example can be achieved via protein-binding molecules linked to oligonucleotides.

This novel assay concept can be implemented - based on the application - into different systems such as, but not limited to, paper-based sensors, microfluidic systems, microarrays, liquid handling platforms, tubes, or microplate-based systems.

Characteristic for the concept is the so called "Nucleic-acid-to-affinity-ligand-transformation" which transforms nucleic acid information into analyte information. This allows the subsequent simultaneous detection of the target nucleic acid and target analyte with the same measuring principle. Additionally, no separate sample preparation is required for the simultaneous detection of the target molecules. For the Nucleic-acid-to-affinity-ligand-transformation an isothermal amplification method is used that simultaneously amplifies the target nucleic acid and introduces adapter molecules (labels) into the nucleic acid. Whereby, the reaction occurs in conditions, which are compatible with analytes such as proteins and thus no separate sample preparation and processing is required. In the subsequent ligand binding assay the labeled target nucleic acid and the target analyte are captured and detected by affinity molecules to generate a signal for the corresponding target molecule.

The invention is based on the finding that nucleic acids and analytes can be analyzed simultaneously in one go from one sample by using Nucleic-acid-to-affinity-ligand-transformation. Furthermore, the invention is based on the finding that analyte compatible isothermal amplification can be used to amplify and label) nucleic acids and subsequently detect both -target analyte and labeled target nucleic acid -simultaneously. Furthermore, the invention is based on the finding that using the same measuring principle allows the detection of all target molecules in one go.

It is preferred that variants of the isothermal amplification are used for analyte compatible amplification of the target nucleic acid and labeling of the target nucleic acid.

In one aspect, the invention provides a method for the simultaneous detection of target analytes and/or target nucleic acids in one go from one sample. Wherein, no separate sample preparation and processing is required for the different target molecule classes and the target nucleic acid is amplified in an analyte compatible isothermal amplification reaction, introducing labels into the amplification product. This process is described as "Nucleic-acid-to-affinity-ligand-transformation". Subsequent, the labeled target nucleic acid and/or the target analyte are detected via ligand binding assay.

The method of the invention is also called Multianalyte Assay and is a novel combination of a Nucleic-acid-to-affinity-ligand-transformation and a ligand binding assay. The Multianalyte Assay of the invention is capable of detecting target analytes and target nucleic acids simultaneously with the same measuring principle.

Nucleic-acid-to-affinity-ligand-transformation describes the functionalization of amplified target nucleic acid sequences with labels to subsequently detect target analyte and target nucleic acid with the same measuring principle. Analyte compatible isothermal amplification is used for the amplification of the target nucleic acid in the presence of the target analyte and the introduction of labels into the amplification product. The labeled amplicon is subsequently detected together with the target analyte via a ligand binding assay. Design parameters of various isothermal amplification reactions are well known to the person skilled in the art.

It is preferred that the second label differs from the first label.

In a preferred embodiment the target nucleic acid is amplified and simultaneously at least the first and the second affinity labels are introduced into the amplified target nucleic acid.
It is preferred, that the primers are about 10 to 80 nucleotides long, especially preferred 20 to 35 nucleotides long and the amplification products should be about 50 to 20000 base pairs long, especially preferred 70 to 500 base pairs long. At least one primer contains an affinity tag (Label 1), preferred at its 5'end.

It is preferred that the first and second label is selected from the group comprising a biotin, FAM, digoxigenin, or dinitrophenyl (DNP), tetramethylrhodamine (TAMRA), texas red, cascade blue, streptavidin or derivatives thereof, Cy5, dansyl, fluorescein, azide, alkyne, or other bio-orthogonal functional groups and/or tags.

It is also preferred that the second affinity label is provided via the second primer and differs from the first label but is also preferred a biotin, FAM, digoxigenin, or dinitrophenyl (DNP), tetramethylrhodamine (TAMRA), texas red, cascade blue, streptavidin or derivatives thereof, Cy5, dansyl, fluorescein, azide, alkyne, or other bio-orthogonal functional groups and/or tags.

One main advantage of the invention is that the detection of a nucleic acid of interest is performed simultaneously with the detection of an analyte of interest. Both, the target analyte and the labeled amplification product (labeled target nucleic acid), are obtained from the above described amplification reaction. Nucleic-acid-to-affinity-ligand-transformation allows the simultaneous detection of the target molecules by the same measuring principle using a ligand binding assay. Here design parameters of a typical ligand binding assay are described. The target analyte and the labeled target nucleic acid are detected in so called detection zones.

A detection zone according to the invention is a defined area of a platform where the detection of the target molecules takes place. A detection zone can be surface but also in solution for example a test line of a lateral flow test, a well of a microtiter plate, a tube, a spot on a microarray, or chamber within a microfluidic device.

In some embodiments the target molecules are detected in the same detection zone. In other embodiments, target analyte and labeled target nucleic acids are detected in different detection zones. In general, the target molecules are captured and/or detected by affinity molecules. Binding of the target molecules to the affinity molecules is typically carried out in buffer solution. This might be a phosphate buffer system or carbonic acid bicarbonate buffer system. Furthermore, additives like blocking agents (e.g. BSA), saccharides (e.g. trehalose) or detergents (e.g. Tween-20) might be added to the buffer system. The person skilled in the art can choose a buffer without being inventive himself. Reaction time and temperature are depending on the target molecules and the used platform. In some embodiments, the detection of the respective target molecules is achieved through attaching signalling tools (e.g. marker) to the affinity molecules. Signalling tools might be, but are not limited to, enzymes, fluorescence reporters, or electrochemiluminescent labels. In other embodiments, the detection of the respective target molecules is achieved through a label-free detection method.

In another preferred embodiment a specific probe is provided which hybridizes with a sequence localized between the at least two amplification primer hybridization sites.

Also preferred is that the specific probe comprises the second affinity label. In this case, the second primer does not need a label.

Also preferred is that the specific probe further comprises at least one functional site, preferred an abasic residue or a polymerase extension blocking group.

In these embodiments, a target nucleic acid (e.g. DNA) is amplified in a sample by binding of at least two primers to the target sequence. At least one of the primers contains an affinity tag (Label 1), preferred at its 5'end. Amplification leads to a primary single labeled (SL) product. This SL product is recognized by a specific probe, which hybridizes to its complementary sequence localized between the two amplification primers. In some embodiments, this probe contains an affinity tag (Label 2) and may further comprise functional sites like abasic residues (e.g. THF) or polymerase extension blocking groups (e.g. C3). Hybridization of the probe to the SL product lead to a second double labeled (DL) product. Typically, this DL product is detected via ligand binding assay.

The amplification is typically carried out after binding of the primers and optionally the probes to the target nucleic acid. Reagents such as nucleotides and DNA polymerase are typically required for the amplification reaction. In some embodiments a DNA polymerase is used that lacks 5' to 3' exonuclease activity. Furthermore, in some embodiments, additional enzymes like a reverse transcriptase can be added to the reaction generate complementary DNA from a RNA template. In some embodiments further components are required for the reaction, this might include, but is not limited to, single-strand binding proteins, recombinases, helicases, or endonucleases. In some embodiments, the addition of magnesium-acetate or other cofactors is required.

If a probe is used, it is preferred that the probe contains the second affinity tag (Label 2), instead of a second labeled primer. The position of the probe is located between the two above described primers. Primers that have the same direction as the probe can overlap.

Readily available software can be used to design primers and/or probes that are complementary to the target nucleic acid. Furthermore, primers and/or probes are designed to avoid areas of secondary structures such as hairpins and stems. The concentration of each primer and/or probe added to the reaction mixture to hybridize to target nucleic acids is typically in the range of 10 to 1000 nM, preferred 50 to 600 nM and 20 to 300 nM.

Further preferred is that the isothermal amplification reaction leads to a primary single labeled product, which is recognized by the specific probe, which hybridizes to a sequence localized between the at least two amplification primers, leading to second DL product.

Any target nucleic acid and/or target analyte can be detected as herein described.

It is also preferred that the target nucleic acid is a DNA molecule, preferably ssDNA, dsDNA, cDNA, rDNA, mtDNA, cpDNA or plasmid DNA a RNA molecule, preferably mRNA, circulating RNA, miRNA, snRNA, snoRNA, rRNA, tRNA, asRNA, circRNA, hnRNA, siRNA, shRNA, snoRNA, snRNA, IncRNA, piRNA, or tracrRNA.

The term "analyte" refers to a substance to be detected, quantified or otherwise assayed by the method of the present invention. Typical analytes may include, but are not limited to proteins, peptides, nucleic acid segments, carbohydrates, lipids, antibodies (monoclonal or polyclonal), antigens, oligonucleotides, specific receptor proteins, ligands, molecules, cells, microorganisms, fragments, products and combinations thereof, or any substance for which attachment sites, binding members or receptors (such as antibodies) can be developed. Analytes may refer also to complexes from said entities. For instance, an analyte may refer to an aggregate or complex formed of multiple entities or molecules, e.g. a protein-protein complex, wherein it is the interaction of the entities/molecules is of interest.

It is especially preferred that the target analyte is a protein, peptide, antibody, hormone, enzyme, small molecule, carbohydrate or any other substance, but not a nucleic acid.

By "protein", a sequence of amino acids is meant for which the chain length is sufficient to produce the higher levels of tertiary and/or quaternary structure. "Peptides" preferably refer to smaller molecular weight proteins.

The term "nucleic acid" refers to any nucleic acid molecule, including, without limitation, DNA, RNA and hybrids or modified variants and polymers ("polynucleotides") thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid molecule/polynucleotide also implicitly encompasses conservatively modified variants thereof (e.g. degenerate codon substitutions) and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19: 5081 (1991); Ohtsuka et al., J. Biol. Chem. 260: 2605-2608 (1985); Rossolini et al., Mol. Cell. Probes 8: 91-98 (1994)). Nucleotides are indicated by their bases by the following standard abbreviations: adenine (A), cytosine (C), thymine (T), and guanine (G).

Further preferred is that the sample is not splitted and no separate assay procedures and/or protocols are required. This approach allows the simultaneous detection of different target molecules classes with the same measuring principle and thus, no splitting of the sample is required. Hence, less sample volume is required, which is crucial for application where only limited sample material might be accessible. In addition, the Multianalyte Assay according to the invention allows a faster and easier detection of the different target molecules, because no individual protocols for target nucleic acids and target analytes have to be pursued. Furthermore, the reaction conditions are defined for the specific detection principle and thus, no lager buffer or temperature changes are required.

Samples comprising a nucleic acid and/or analyte of interest can be obtained from any source. This might include sources like bacteria, viruses, fungi, organelles, plants, or mammals. Other samples might be obtained from environmental sources and production products (e.g. food or drugs), or bodily fluids (e.g. blood, urine, wound fluid, serum). In typical embodiments, the target nucleic acid is a DNA molecule (e.g. ssDNA, dsDNA, cDNA). In other embodiments, the target nucleic acid is a RNA molecule (e.g. mRNA, circulating RNA, miRNA). The targets can include for example nucleic acids associated with pathogens (e.g. bacteria, viruses, or fungi), with diseases (e.g. over- and under-expression) or nucleic acid based therapeutics. In typical embodiments, the target analyte is a protein. In other embodiments, the target analyte is a peptide, antibody, hormone, small molecule carbohydrate or any other substance, but not a nucleic acid. In some embodiments the analyte might be associated with inflammation (e.g. interleukin, CRP), diseases, allergens, therapeutic drugs, or regulatory function. In general, the invention comprises the simultaneous detection of target nucleic acids and target analytes in one go from one sample using the herein described Multianalyte Assay. This includes, analyte compatible Nucleic-acid-to-affinity-ligand-transformation together with a ligand binding assay. Thus, this approach does not require separate sample preparation and processing. Target nucleic acid and target analyte can be added directly simultaneously to the amplification reaction. This is possible, due to the analyte compatibility of the amplification reaction. Furthermore, the target molecules are detected with the same measuring principle using a ligand binding assay.

It is preferred that the detection of a target nucleic acid of interest, is performed simultaneously with the detection of an analyte of interest thus the amplification reaction conditions are compatible with the analyte stability. Time and temperature of the amplification reaction can vary, depending on primers, probes (if present) and the target analyte. Preferred, the reaction requires 10 min to 2 hours at RT to 45 °C.

Therefore, it is preferred that the isothermal amplification reaction occurs in conditions, which are compatible with analytes. In general, this means that the reaction conditions (e.g. temperature, buffer) have to be chosen in a way that maintain an adequate analyte structure such that affinity molecules are able to bind the analyte. Therefore, e.g. the buffer has to be liquid and the analytes must not be denatured, so the temperature needs to be between 0 and 100°C. The conditions depend on the analytes as well as on the selected amplification method and the ligand binding assay. The person skilled in the art can choose suitable parameters without being inventive himself. For temperature sensitive analytes, e.g. heat unstable proteins, an amplification method must be selected that can be performed at low temperatures (25 - 40 °C), e.g. recombinase polymerase amplification (RPA). Other analytes are also stable at higher temperatures and e.g. a Loop-mediated isothermal amplification (LAMP) can be performed at 40 - 70°C. Also, there are analytes which allow an initial denaturation step (80 - 100 °C) prior to isothermal amplification, e.g. to melt dsDNA.

Amplification is usually carried out in the neutral to slightly basic (pH 7.0 - 8.3) pH range. This is compatible with most analytes. However, if necessary, the pH value can also be reduced to the slightly acidic range (pH 5.0 - 7.0). Other pH values are possible as well and the person skilled in the art knows which pH range is compatible with what kind of analyte, e.g. LAMP reactions can occur within a pH range of 6.0-10.0 or HAD within a range of about pH 6.0-9.0 (buffer of Tris-acetate or Tris-HCI).

It is further preferred that the signal for the target nucleic acid is generated via the binding of affinity labels by affinity molecules. Affinity molecules are therefore molecules that bind affinity labels according to the invention. Therefore, affinity molecules are preferred molecules containing a target binding moiety which might bind to a target analyte or a labeled target nucleic acid.

Affinity molecules are also used to bind and detect the target analytes. The term "affinity molecule" preferably refers to one member of a binding pair, wherein the second member is the analyte and/or the affinity label and wherein the term "binding pair" includes any of the class of immune-type binding pairs, such as antigen/antibody or hapten/anti-hapten systems; and also any of the class of nonimmune-type binding pairs, such as biotin/avidin; biotin/streptavidin; folic acid/folate binding protein; complementary nucleic acid segments such as complementary DNA strands or complementary RNA strands; protein A or G/immunoglobulins; and binding pairs which form covalent bonds, such as sulfhydryl reactive groups including maleimides and haloacetyl derivatives, and amine reactive groups such as isotriocyanates, succinimidyl esters and sulfonyl halides. A polymer matrix, like molecularly imprinted polymers can also be affinity molecules according to the invention

It is preferred that the affinity molecules are antibodies and/or proteins, and/or aptamers and/or functional groups and/or ligand binding polymer structures and/or molecularly imprinted polymers (MIPs) and/or (macro-) molecules which can contain a functional group and/or wherein signalling tools, preferred marker are attached to the affinity molecules.

Any isothermal amplification method, that has target analyte compatible reaction condition, can be used for the method of the invention. The described amplification reactions are just examples and shall not limit the invention.

In some embodiments, recombinase polymerase amplification (RPA) might be used to amplify the target nucleic acid. The RPA is an isothermal amplification method that operates at 37 to 42 °C. Instead of temperature cycling the method uses proteins which are involved in cellular DNA synthesis, recombination and repair. In the presence of ATP and a crowding agent a recombinase protein bind to primers and promotes strand invasion at homologous sequences. The displaced strand is stabilized by single-strand binding proteins. A strand displacing DNA polymerase is responsible for the elongation of the primer. Cycling of the process results in exponential amplification of the target nucleic acid. In some embodiments a reverse-transcription RPA is used. In other embodiments, an additional specific probe is used.

In this specific embodiment, it is preferred that one set of primers is designed to amplify the target nucleic acid. At least one primer contains an affinity tag (label 1), preferred at its 5'end. The affinity tag might be a biotin, FAM, digoxigenin, or DNP. In general, RPA primers are preferred 30 to 35 nucleotides long and the amplification product can be about 70 to 500 base pairs long. To increase the specificity a specific probe may be added. Typically, the probe is about 46-53 nucleotides long, at least 30 nucleotides of which are placed 5' of the abasic side, and at least a further 15 nucleotides are located 3' to it. The affinity tag (Label 2) is located at the 5'end and might be a biotin, FAM, digoxigenin, or DNP (should differ from Label 1). Whereas, the polymerase extension blocking group is located at the 3'end and might be a phosphate, C3-spacer or a dideoxy nucleotide. The position of the probe is located between the two above described primers. Primers that have the same direction as the probe can overlap. However, it is preferred that the overlap is restricted to the first 30 nucleotides of the probe. The concentration of each primer and probe added to the reaction mixture to hybridize to target nucleic acids is typically in the range of 150 to 600 nM and 50 to 300 nM.

In some embodiments, helicase-dependent amplification (HDA) might be used for the amplification of target nucleic acid. In general, the HDA uses a helicase and single-strand binding proteins to generate single-stranded templates for primer hybridization. A strand-displacing DNA-Polymerase extend the primers and produces a dsDNA. Cycling of the process results in exponential amplification of the target nucleic acid. In some embodiments a reverse-transcription HDA is used.

In this specific embodiment one set of primer is designed to amplify the target nucleic acid. At least one primer contains an affinity tag as described before. Preferably the primers are 24 to 33 nucleotides long and the amplification product can be about 80 to 129 base pairs long. To increase the specificity a specific probe may be added to the reaction. The probe is labeled with an affinity tag (Label 2), which is located at the 3'end to avoid extension and might be a biotin, FAM, digoxigenin, or DNP (should differ from Label 1). The position of the probe is located between the two above described primers. Whereby, the non-labeled primer is limiting the reaction. The concentration of each primer, added to the reaction mixture, is typically in the range of 50 to 200 nM.

In some embodiments, strand displacement amplification (SDA) might be used for the amplification of target nucleic acid. The SDA is based on the nicking activity of a restriction enzyme and on the ability of the DNA polymerase to initiate replication at a nick and displace the downstream sequence. In a typical SDA, two primer sets are designed that bind to the target nucleic acid - two outer "bumper" primers and two inner extension primers containing a nicking enzyme recognition site. A variation of the SDA might use the CRISPR-Cas system, wherein a pair of Cas9 ribonucleoproteins recognize the target DNA and induce a pair of nicks. To increase the specificity, two probes, that target two different regions of the amplicon, might be added to the reaction. The capture probe is labeled with an affinity tag at the 5'end (Label 1), whereas the detection probe is labeled with an affinity tag at the 3'end (Label 2). The affinity tag might be a biotin, FAM, digoxigenin, or DNP.

However, these described amplification reactions are just examples. In other embodiments, isothermal amplification methods such as Loop-mediated isothermal amplification (LAMP), or nucleic acid sequence-based amplification (NASBA), might be used. In addition, an internal amplification control (IAC) nucleic acid might be amplified simultaneously with the target nucleic acid to exclude false negative results.

The assay includes besides the isothermal amplification a ligand binding assay. A ligand binding assay is an analytical procedure for the detection of target molecules, which relies on the binding of target molecules to affinity molecules. This interaction/binding can be covalent or non-covalent.

A ligand binding assay is an assay, which relies on the covalent or non-covalent binding of target molecules (ligands) to affinity molecules. In a first embodiment an antigen-antibody reaction is used for the simultaneous detection of the target molecules. Whereas another embodiment describes the usage of aptamers. It is also possible to use both variants simultaneously. In other embodiments, different covalent or non-covalent ligand binding concepts (e.g. ligand binding polymer structures) might be implemented.

The usage of antigen-antibody reactions for the detection of analytes is state of the art. The antibody recognizes the target analyte and can bind to its epitope. Also, the affinity labels of the DL amplicon can be recognized by specific antibodies. Various immunoassay formats such as competitive immunoassay or sandwich immunoassay can be used for the detection of target analytes and labeled target nucleic acids.

Aptamers are oligonucleotide-based affinity molecules that bind to target analytes. They have sufficient surface areas to recognize and bind their targets and can differentiate between isoform and variants of a target analyte (like monoclonal antibodies). Various immunoassay formats such as competitive immunoassay or sandwich immunoassay can be used for the detection of target molecules via aptamers.

All required components of the Multianalyte Assay are commercially available and the assay requires no complex design steps (e.g. functionalization of antibodies with oligonucleotide sequences or design of complex primer and probe systems).

It is further preferred that the method is integrated into a platform. Preferred the platform is a microtiter plate, a lateral flow test, an array based platform, especially preferred a microarray, a microfluidic platform, preferred Lab-on-a-Chip or bigger systems or liquid handling platforms, or any other suitable platform.

The use of microtiter plate assays is a conventional and straightforward approach. This platform can be used in laboratories for example to perform high-throughput protein expression screening. The use of pipetting robots can automatize this process. In a specific embodiment, the sample is added to a first well. Within this first well, the pervious described nucleic-acid-to-affinity-ligand-transformation reaction takes place. Subsequently, a certain amount of the nucleic-acid-to-affinity-ligand-transformation reaction is transferred to a second well (detection zone) to perform the ligand binding assay. The target molecules are captured and/or detected by affinity molecules which generates a e.g. optical signal for the respective target molecule. Finally, a commercial microtiter plate reader performs the optical readout of the plate.

Lateral flow tests (LFT) are paper-based devices that allow low-cost, simple, rapid and portable detection of target molecules. LFTs are widely used in different industries including, diagnostics, quality control, product safety in food production, and environmental health and safety. In general, the sample moves via capillary force through various zones of the lateral flow strip. In some embodiments, the nucleic-acid-to-affinity-ligand-transformation reaction takes place within a tube or other cavity and subsequently a certain amount of the reaction is transferred onto the lateral flow strip. Whereas, in other embodiments, the nucleic-acid-to-affinity-ligand-transformation reaction is integrated into the lateral flow strip. The subsequent ligand binding assay is integrated into the lateral flow strip. An affinity molecule (e.g. functionalized gold nanoparticle) binds to the target molecules and the complex is captured by a second affinity molecule (e.g. antibody) to the detection zone (e.g. test line). Subsequently, the signal for the corresponding molecule is detected via optical read out by the naked eye. In further embodiments, the read out might be based on electrochemical or fluorescence detection, or other detection formats known by the person skilled in the art, e.g. photoacoustic, etc.

Microarrays are high-throughput platforms and the parallel detection of multiple target molecules is their main advantage. To simultaneously detect target nucleic acids and targets analytes one amplification primer and the capture molecule for the target analyte might be immobilized on the detection zones. After adding a mixture of sample and nucleic-acid-to-affinity-ligand-transformation reaction components to the microarray the device incubated for a sufficient time. In other embodiments, the nucleic-acid-to-affinity-ligand-transformation reaction can take place within a separated reaction chamber and subsequently a certain amount of the reaction is transferred to the detection zone of the microarray. After the addition of the detection molecule the corresponding signal is detected.

Microfluidic platforms provide a set of fluidic unit operations, which allows the miniaturization, integration, automation and parallelization of biochemical assays. Possible microfluidic platforms include, but are not limited to, pressure driven microfluidics, centrifugal microfluidics, electrokinetic microfluidics, and "microfluidic large scale integration" approaches. All reagents for the Multianalyte Assay can be pre-stored within the microfluidic platform. After adding the sample, all processing steps are carried out by the microfluidic platform. The pre-stored reagents are transported into reaction/detection chambers, which allows automated nucleic-acid-to-affinity-ligand-transformation and subsequent detection of the target molecules via ligand binding assay.

In another preferred embodiment the invention relates to a kit for performing a method according to any one or more of the preceding claims, the kit comprising
- at least one set of amplification primers, which hybridize with a target nucleic acid,
- wherein at least one of the amplification primers comprises a first affinity label,
- optionally a specific probe,
- a second affinity label which is either associated with the second primer or with a specific probe, which can hybridize with the target nucleic acid,
- reagents for performing an isothermal amplification reaction and
- affinity molecules.

All preferred embodiments described for the method a preferred embodiments of the kit as well.

Preferred the kit comprises a solution in which an amplification reaction takes place and may include but is not limited to polymerase, recombinase, endonuclease, amplification reagents, amplicons, buffering agents, cations, dNTPs, and/or other components.

In another preferred embodiment the invention relates to the use of the method or the kit according to any one or more of the preceding claims for in-vitro diagnostics, drug development, food safety, or environmental safety.

The invention is advantageous especially in drug development, where only limited sample material might be accessible. A simultaneous detection of nucleic acids and analytes enables the analysis of gene expression modulation along with the corresponding analyte product. In terms of food safety, a simultaneous detection of analytes and nucleic acids is also very beneficial as microbial contaminants can be detected along with allergenic ingredients, toxins or other biomolecular contaminants.

The described invention can be used to predict or detect a disease, or determining predisposition to the disease, or monitoring the treatment of the disease, or diagnosing the therapeutic response.

A wide variety of infectious diseases can be detected by the process of the present invention. Typically, these are caused by bacteria, parasite, fungi or viruses. The invention can be used to identify pathogens along with inflammatory protein markers of a host for the detection of infectious diseases. This is especially beneficial for fast therapy decision in time-critical infections, or for monitoring the status of the infection.

An example is the simultaneous detection of bacterial genomic DNA (e.g. *P. aeruginosa* gDNA) together with inflammatory cytokine marker (e.g. interleukin-6). The pathogen and the cytokine might be detected in bodily fluids like wound fluid or sputum. In this specific embodiment, the bacterial DNA is amplified in the presence of the cytokine and subsequently the target molecules are detected via ligand binding assay.

In other embodiments, the invention can be used to detect viral RNA together with specific antibodies against the virus or specific virus antigens to increase the reliability of a test. Using a reverse transcription, the RNA is transcribed into cDNA and is subsequently amplified and simultaneously detected with the specific antibodies/antigens via Multianalyte Assay. The virus might be detected together with the specific antibody/antigen in bodily fluids like cerebrospinal fluid, sputum, or other swab samples.

In other embodiments, the invention can be used for the diagnosis or staging of a cancer. A bodily fluid like blood might be used to determine the presence and/or quantity of tumor markers. The invention allows the simultaneous detection of target analytes like proteins, hormones and enzymes specific for a cancer type or stage together with target nucleic acids like circulating micro RNAs or ctDNA. This allows for example early-stage diagnosis, guidance for therapeutic strategies and monitoring of the cancer status.

The Multianalyt Assay of the invention can also be used to screen for drugs, e.g. drug development, or screening of targets for pharmaceutical development. A simultaneous detection of nucleic acids and analytes enables the analysis of pathways and gene expression modulation along with the corresponding protein.

The Multianalyt Assay of the invention can also be used in the field of food safety. In terms of food safety, a simultaneous detection of analytes and nucleic acids could be very beneficial as microbial contaminants could be detected along with allergenic ingredients, toxins or other biomolecular contaminants.

One major advantage of the presented invention is the flexibility of the approach.

Another advantage is that all required components of the Multianalyte Assay are commercially available and that the assay requires no complex design steps (e.g. functionalization of antibodies with oligonucleotide sequences or design of complex primer and probe systems). Since the assay is easy to implement and can be customized flexibly to specific needs, a quick integration is possible.

The present invention introduces a new dimension of multiplexing and enables multiplexing for different molecules classes and allows the detection of all molecules that can be detected via ligand binding assays.

The invention allows for fast and easy adaption of the technology on large scale laboratory automates as well as its integration into small and specialized point-of-care devices.

The invention (method, kit and use) is advantageous in many applications. To detect several classes, preferred at least two, of molecules simultaneously in a single step, has been a long-desired goal. The invention can be used to identify pathogens with a DNA analysis along with inflammatory protein markers of a host for the detection of infectious diseases. The invention has a huge impact especially for fast therapy decision in time-critical infections, since there is no time to follow different assay protocols and analysis methods. With the novel approach of the invention a bioanalytical result with all important information on the pathogen and host inflammation is available much faster compared to the state of the art.

All cited documents of the patent and non-patent literature are hereby incorporated by reference in their entirety.

### Figures and examples

Before the present invention is described with regards to the examples, it is to be understood that the terminology used herein is for the purpose of describing particular embodiments only and is not intended to limit the scope of the present invention.

### Example: Simultaneous detection of bacterial genomic DNA together with inflammatory cytokine marker.

The simultaneous detection of pathogen and inflammatory cytokine enables the simultaneous consideration of infection and host response from one sample. This is especially beneficial for fast therapy decision in time-critical infections, for monitoring the status of the infection, or a personalized therapy. As an example, the simultaneous detection of genomic DNA (gDNA) of *Pseudomonas aeruginosa* (*P*. *aeruginosa*) in combination with the acute marker interleukin-6 (IL-6) is shown.

Nucleic-acid-to-affinity-ligand-transformation describes the functionalization of amplified target nucleic acid sequences with labels to subsequently detect target analyte and target nucleic acid with the same measuring principle. Thus, our Multianalyte Assay is capable of detecting target analytes and target nucleic acids simultaneously with the same measuring principle. In this particular example, RPA is used to amplify and label the gDNA of *P*. *aeruginosa* in the presence of IL-6. Subsequently, both analytes are detected via sandwich immunoassay within a well of a microtiter plate.

Primer and probes in this specific example are designed to target a highly conserver region of the LasB gene of *P*. *aeruginosa.* The sequence of the used primers and probe are indicated in Table 1. The set of LasB specific primers (LasB-fwd and LasB-rev primer) and probe (LasB-probe) produce a single-labeled (digoxigenin) 161 bp sized product and a double-labeled (digoxigenin and biotin) 123 bp sized product. Only the double-labeled product is detected via ligand binding assay.

The RPA is performed in a 50 µl volume using the TwistAmp^{®} nfo kit (TwistDx). Briefly, 29.5 µl 1x rehydration buffer was mixed with 1.25 µl LasB-fwd primer (10 µM), 1.25 µl LasB-rev primer (10 µM), and 1.2 µl LasB-probe (10 µM). Subsequently, 6 µl sample (containing genomic DNA and IL-6) and 8.3 µl ddH2O are added to the reaction mix. Next, the reaction pellet and 2.5 µl of magnesium acetate (280 nM) are added and the tubes are placed immediately onto a block heater for 15 min at 37 °C.

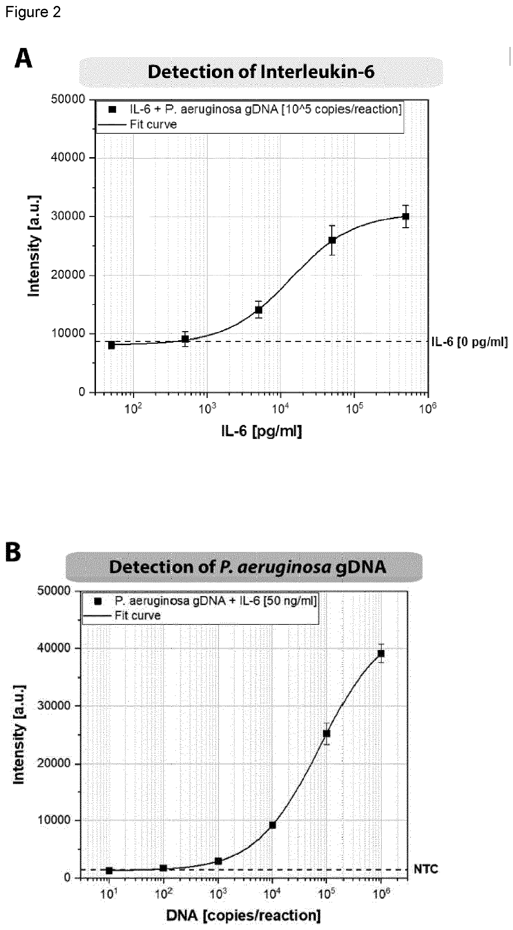

Subsequently, the RPA reaction is diluted 1:10 in 100 µl assay buffer (1xPBS, 0.1% BSA) and added to a microtiter plate coated with 4 µg/ml anti-IL6 antibody and 4 µg/ml streptavidin. The microtiter plate is blocked previously with PBS containing 5 % BSA. After incubating the labeled target DNA and the target analyte for 1 hour at RT, the microtiter plate is washed three times with PBS containing 0.05 % Tween 20. Subsequently, the target molecules are detected via anti-digoxigenin-conjugated fluorescent microspheres and anti-IL6-conjugated fluorescent microspheres. Therefore, 100 µl of each conjugated fluorescent microsphere (75 µg/ml) are added to the microtiter plate and incubated for 1 hour at RT. Again, the microtiter plate is washed three times with PBS containing 0.05 % Tween 20. Finally, 100 µl of PBS are added to the microtiter plate and the fluorescent signal for each target molecule is detected via commercial microtiter plate reader (Figure 2).

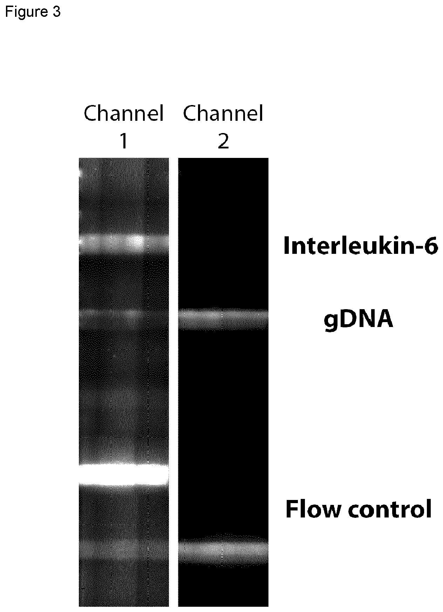

In a different example IL-6 and *P*. *aeruginosa* gDNA are detected via lateral flow assay (Figure 3).

**Table 1**

| **Name** | **Sequence (5'-3')** |
|---|---|
| LasB-fwd primer | GAGAATGACAAAGTGGAACTGGTGATCCGCCTG |
| LasB-rev primer | Diq-GCCAGGCCTTCCCACTGATCGAGCAC TTCGCCG |
| LasB-probe | Biotin-GAACAACATCGCCCAACTGGTCTACAACGT[H]TCCTACCTG ATTCCC-C3 spacer |
| Dig, Digoxigenin; | H, tetrahydrofuran; C3 spacer, polymerase extension blocking group |

Table 1 shows primer and probe sequences described for the detection of the simultaneous detection of *P*. *aeruginosa* gDNA and IL-6.
SEQ ID No. 1 GAGAATGACAAAGTGGAACTGGTGATCCGCCTG
SEQ ID No. 2 GCCAGGCCTTCCCACTGATCGAGCAC TTCGCCG

SEQ ID No. 3 GAACAACATCGCCCAACTGGTCTACAACGTTCCTACCTG
ATTCCC

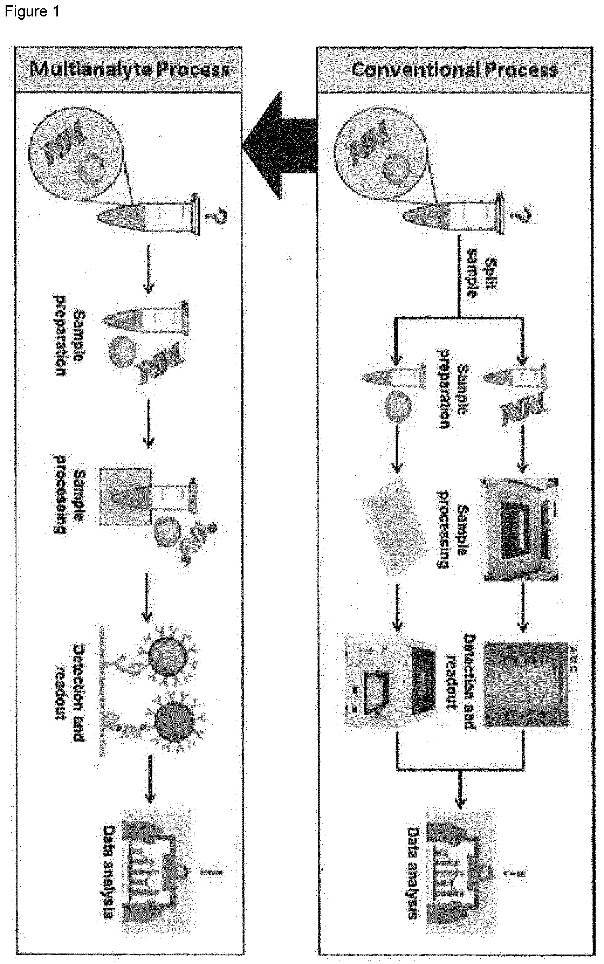

**Figure 1** shows a conventional detection of multiple target molecules versus simultaneous detection of multiple target molecules in one go from one sample. (Top) Conventional detection of multiple target molecules. (Bottom) Simultaneous detection of the target molecules via Multianalyte Assay according to the invention.
**Figure 2** shows the simultaneous detection of *P*. *aeruginosa* gDNA and IL-6 within within a microtiter plate.
**Figure 3** shows the simultaneous detection of P. aeruginosa gDNA and IL-6 via lateral flow assay.

### References

Cook, Damon B.; McLucas, Brian C.; Montoya, Leticia A.; Brotski, Chris M.; Das, Shelley; Miholits, Markus; Sebata, Thao H. (2019): Multiplexing protein and gene level measurements an a single Luminex platform. In: Methods (San Diego, Calif.) 158, S. 27-32. DOI: 10.1016/j.ymeth.2019.01.018.
Darmanis, Spyros; Gallant, Caroline Julie; Marinescu, Voichita Dana; Niklasson, Mia; Segerman, Anna; Flamourakis, Georgios et al. (2016): Simultaneous Multiplexed Measurement of RNA and Proteins in Single Cells. In: Cell reports 14 (2), S. 380-389. DOI: 10.1016/j.celrep.2015.12.021.
Delley, Cyrille L.; Liu, Leclian; Sarhan, Maen·F.; Abate, Adam R. (2018): Combined aptamer and transcriptome sequencing of single cells. In: Scientific reports 8 (1), S. 2919. DO1: 10.1038/s41598-01821153-y.
Frei, Andreas P.; Bava, Felice-Alessio; Zunder, Eli R.; Hsieh, Elena W. Y.; Chen, Shih-Yu; Nolan, Garry P.; Gherardini, Pier Federico (2016): Highly multiplexed simultaneous detection of RNAs and proteins in single cells. In: Nat Methods 13 (3), S. 269-275. DOI: 10.1038/nmeth.3742.
Geiss, Gary K.; Bumgamer, Roger E.; Birditt, Brian; Dahl, Timothy; Dowidar, Naeem; Dunaway, Dwayne L. et al. (2008): Direct multiplexed measurement of gene expression with color-coded probe pairs. In: Nature biotechnology 26 (3), S. 317-325. DO1: 10.1038/nbt1385.
Gong, Haibiao; Wang, Xiaohui; Liu, Benjamin; Boutet, Stephane; Holcomb, Ilona; Dakshinamoorthy, Gajalakshmi et al. (2017): Single-cell protein-mRNA correlation analysis enabled by multiplexed dual-analyte co-detection. In: Scientific reports 7 (1), S. 2776. DOI: 10.1038/s41598-017-03057-5.
Katja Niemann, Vicky Troger (2015): Isothermal Amplification and Quantification of Nucleic Acids and its Use in Microsystems. In: J Nanomed Nanotechnol 06 (03). DOI: 10.4172/2157-7439.1000282.
Mao, Xun; Gurung, Anant; Xu, Hui; Baloda, Meenu; He, Yuqing; Liu, Guodong (2014): Simultaneous Detection of Nucleic Acid and Protein Using Gold Nanoparticles and Lateral Flow Device. In: Analytical sciences: the international Journal of the Japan Society for Analytical Chemistry 30 (6), S. 637-642.
Mohan, Ruchika; Mach, Kathleen E.; Bercovici, Moran; Pan, Ying; Dhulipala, Lakshmi; Wong, Pak Kin; Liao, Joseph C. (2011): Clinical validation of integrated nucleic acid and protein detection on an electrochemical biosensor array for urinary tract infection diagnosis. In: PloS one 6 (10), e26846. DOI: 10.1371/journal.pone.0026846.
Naik, Priyanka; Manna, Riddha; Paul, Debjani (2019): Nucleic Acid Amplification on Paper Substrates. In: Shantanu Bhattacharya, Sanjay Kumar und Avinash K. Agarwal (Hg.): Paper Microfluidics, Bd. 43. Singapore: Springer Singapore (Advanced Functional Materials and Sensors), 5. 115-146.
Ooi, Aik T.; Ruff, David W. (2019): Simultaneous Targeted Detection of Proteins and RNAs in Single Cells. In: Methods in molecular biology (Clifton, N.J.) 1979, S. 379-392. DOI: 10.1007/978-1-4939-9240-9_22.
Peterson, Vanessa M.; Zhang, Kelvin Xi; Kumar, Namit; Wong, Jerelyn; Li, Lixia; Wilson, Douglas C. et al. (2017): Multiplexed quantification of proteins and transcripts in single cells. In: Nature biotechnology 35 (10), S. 936-939. D01: 10.1038/nbt.3973.
Schulz, Daniel; Zanotelli, Vito Riccardo Tomaso; Fischer, Jana Raja; Schapiro, Denis; Engler, Stefanie; Lun, Xiao-Kang et al. (2018): Simultaneous Multiplexed Imaging of mRNA and Proteins with Subcellular Resolution in Breast Cancer Tissue Samples by Mass Cytometry. In: Cell systems 6 (1), 25-36.e5. DOI: 10.1016/j.cels.2017.12.001.
Scott, Alexander W.; Garimella, Viswanadham; Calabrese, Colin M.; Mirkin, Chad A. (2017): Universal Biotin-PEG-Linked Gold Nanoparticle Probes for the Simultaneous Detection of Nucleic Acids and Proteins. In: Bioconjugate chemistry 28 (1), S. 203-211. DO1: 10.1021/acs.bioconjchem.6b00529.
Stoeckius, Marion; Hafemeister, Christoph; Stephenson, William; Houck-Loomis, Brian; Chattopadhyay, Pratip K.; Swerdlow, Harold et al. (2017): Simultaneous epitope and transcriptome measurement in single cells. In: Nature methods 14 (9), 5. 865-868. DO1: 10.1038/nmeth.4380.
Todorovic, Vesna (2017): Single-cell RNA-seq-now with protein. In: Nat Methods 14 (11), 5. 1028-1029. DOI: 10.1038/nmeth.4488.
Ullal, Adeeti V.; Peterson, Vanessa; Agasti, Sarit S.; Tuang, Suan; Juric, Dejan; Castro, Cesar M.; Weissleder, Ralph (2014): Cancer cell profiling by barcoding allows multiplexed protein analysis in fine-needle aspirates. In: Science transiational medicine 6 (219), 219ra9. D01: 10.1126/scitranslmed.3007361.
Warren, Sarah (2018): Simultaneous, Multiplexed Detection of RNA and Protein an the NanoString® nCounter® Platform. In: Methods in molecular biology (Clifton, N.J.) 1783, S. 105-120. D01: 10.1007/9781-4939-7834-2_5.

## Claims

1. Method to detect the presence or absence of at least two different target molecules form one sample, wherein at least one target molecule is a target analyte of interest and at least one other target molecule is a target nucleic acid of interest, the method comprising
• performing an isothermal amplification reaction, comprising
∘ contacting a sample to be analyzed for the presence or absence of at least one target nucleic acid and/or at least one target analyte to at least one set of amplification primers,
∘ wherein the two amplification primers can hybridize with the target nucleic acid,
∘ wherein at least one of the amplification primers comprises a first affinity label,
∘ wherein a second affinity label is provided in a way that in can be incorporated into an amplicon of the target nucleic acid,
• simultaneously performing a ligand binding assay, wherein affinity molecules are used, which can capture and detect the presence of target analytes and/or labeled target nucleic acid via signal generation,
• detecting the presence or absence of said target analytes and/or target nucleic acids.

2. Method according to claim 1, wherein the target nucleic acid is amplified and simultaneously at least the first and the second affinity labels are introduced into the amplified target nucleic acid.

3. Method according to claim 1 or 2, wherein a specific probe is provided which hybridizes with a sequence localized between the at least two amplification primer hybridization sites, and
wherein the specific probe comprises the second affinity label.

4. Method according to the preceding claim, wherein the isothermal amplification reaction leads to a primary single labeled product, which is recognized by the specific probe, which hybridizes to a sequence localized between the at least two amplification primers, leading to second double labeled product.

5. Method according to any one or more of the preceding claims,
wherein the second label differs from the first label.

6. Method according to any one or more of the preceding claims, wherein the signal for the target nucleic acid is generated via the binding of affinity labels by affinity molecules.

7. Method according to any one or more of the preceding claims, wherein the target nucleic acid is
a DNA molecule, preferably ssDNA, dsDNA, cDNA, rDNA, mtDNA, cpDNA or plasmid DNA
a RNA molecule, preferably mRNA, circulating RNA, miRNA, snRNA, snoRNA, rRNA, tRNA, asRNA, circRNA, hnRNA, siRNA, shRNA, snoRNA, snRNA, IncRNA, piRNA, or tracrRNA .

8. Method according to any one or more of the preceding claims, wherein the target analyte is a protein, peptide, antibody, hormone, enzyme, small molecule, carbohydrate or any other substance, but not a nucleic acid.

9. Method according to any one or more of the preceding claims, wherein the sample is not splitted and no separate assay procedures and/or protocols are required.

10. Method according to any one or more of the preceding claims, wherein the isothermal amplification reaction occurs in conditions, which are compatible with analytes.

11. Method according to any one or more of the preceding claims, wherein the first label is an affinity tag, preferred a biotin, FAM, digoxigenin or dinitrophenyl (DNP), tetramethylrhodamine (TAMRA), texas red, cascade blue, streptavidin and derivatives, Cy5, dansyl, fluorescein, azide, alkyne, or other bio-orthogonal functional groups and/or tags.

12. Method according to any one or more of the preceding claims 3 to 11, wherein the specific probe further comprises at least one functional site, preferred an abasic residue or a polymerase extension blocking group.

13. Method according to any one or more of the preceding claims, wherein the affinity molecules are antibodies, aptamers, functional groups, proteins, ligand binding polymer structures, (macro-) molecules which can contain a functional group and/or molecularly imprinted polymers (MIPs) and/or wherein signalling tools, preferred marker are attached to the affinity molecules.

14. Kit for performing a method according to any one or more of the preceding claims, the kit comprising
• at least one set of amplification primers, which hybridize with a target nucleic acid,
• wherein at least one of the amplification primers comprises a first affinity label,
• optionally a specific probe, which can hybridize with the target nucleic acid,
• a second affinity label which is either associated with the second primer or with the specific probe,
• reagents for performing an isothermal amplification reaction and
• affinity molecules.

15. Use of the method or the kit according to any one or more of the preceding claims for in vitro diagnostics, drug development, food safety, or environmental safety.


SEQUENCE LISTING

<110>  Albert-Ludwigs-Universität Freiburg
       Hahn-Schickard-Gesellschaft für angewandte Forschung e.V.

<120>  Multianalyte Assay for the simultaneous detection of nucleic acid
        and analytes

<130>  XII2184/20EP

<160>  3

<170>  PatentIn version 3.5

<210>  1
<211>  33
<212>  DNA
<213>  Artificial Sequence

<220>
<223>  primer

<400>  1
gagaatgaca aagtggaact ggtgatccgc ctg                                    33


<210>  2
<211>  33
<212>  DNA
<213>  Artificial Sequence

<220>
<223>  primer

<400>  2
gccaggcctt cccactgatc gagcacttcg ccg                                    33


<210>  3
<211>  45
<212>  DNA
<213>  Artificial Sequence

<220>
<223>  probe

<400>  3
gaacaacatc gcccaactgg tctacaacgt tcctacctga ttccc                       45